# EUROPEAN PATENT SPECIFICATION

(11) **EP 2 484 397 B1**
(45) Date of publication and mention of the grant of the patent: **21.01.2015**
(21) Application number: 10820340.7
(22) Date of filing: 13.09.2010
(51) Int. Cl.: A61M 5/32

(54) **SYRINGE NEEDLE ASSEMBLY AND MEDICATION SYRINGE DEVICE**
SPRITZENNADELANORDNUNG UND MEDIKAMENTENSPRITZENVORRICHTUNG
ENSEMBLE AIGUILLE DE SERINGUE ET DISPOSITIF DE SERINGUE POUR MÉDICAMENT

(30) Priority: 30.09.2009 JP 2009228548
(43) Date of publication of application: 08.08.2012
(73) Proprietor: Terumo Kabushiki Kaisha, Tokyo 151-0072 (JP)
(72) Inventor: YOKOTA Takayuki, Nakakoma-gun Yamanashi 409-3853 (JP); IWASE Yoichiro, Ashigarakami-gun Kanagawa 259-0151 (JP); HISHIKAWA Yoshinori, Nakakoma-gun Yamanashi 409-3853 (JP); YAGISHITA Eiji, Nakakoma-gun Yamanashi 409-3853 (JP)
(74) Representative: TBK
(86) International application number: PCT/JP2010/065746
(87) International publication number: WO 2011/040221

(56) References cited:
- WO-A1-02/47550
- WO-A1-2004/000118
- WO-A1-2007/111519
- GB-A- 2 352 403
- JP-A- 8 000 597
- JP-A- 2008 307 423
- US-A1- 2007 118 077
- US-B1- 6 589 261

## Description

The present invention relates to an injection needle assembly (i.e., a syringe needle assembly) according to the preamble of claim 1 and a drug injection device (i.e., a medication syringe device) using the same.

### Background Art

Typically, an injection needle assembly includes a needle tube whose tip-end portion having a needle tip capable of puncturing a living body, and a needle hub adapted to hold the needle tube in a state where the needle tip of the needle tube is protruded. Further, a drug injection device is configured by connecting a syringe to the needle hub. Conventionally, the needle tube is fixed to the needle hub by using an adhesive in a state where the needle tube is inserted through an insertion hole of the needle hub (see, for example, JP-A-2006-116163).

In recent years, in order to reduce the time for performing the process of adhering the needle tube and the needle hub to each other, it is considered to integrally form the needle hub and the needle tube by insert molding in a state where the needle tube is held by a chuck member.

However, when molding the needle hub around the needle tube by insert molding, it is necessary to hold the needle tube. Thus, if the length of the needle tube protruded from the needle hub is very small, it will be difficult to hold and fix the needle tube with a chuck member when performing injection molding and assembling. Further, even in the case where the needle tube can be held at both ends on the axial direction thereof, if the needle tube is small in diameter, there will be a concern that the needle tube might be deformed and/or bent due to the molding pressure generated when molding. Thus, in a conventional injection needle assembly, since the needle tube can not be reliably held and fixed by a chuck member, the needle tube and the needle hub can not be integrally formed by insert molding.

US 2007/118077 A1 shows a generic injection needle assembly according to the preamble of claim 1. The injection needle assembly comprises a needle tube having a needle tip capable of puncturing a living body; and a needle hub that is adapted to hold the needle tube in a state where the needle tip of the needle tube is protruded, wherein the needle hub has a stabilizer arranged to cover around the needle tube in a state where the needle tip of the needle tube is protruded, the stabilizer having an end face which contacts the skin when the needle tube punctures the living body.

WO 2004/000118 A1, GB 2 352 403 A, WO 02/47550 A1 or US 6 589 261 B1 disclose an injection needle assembly having a needle hub for holding a needle tube, in which needle hub respective retention holes are provided at a base end portion of the hub opposite to the tip end of the needle tube.

### SUMMARY OF THE INVENTION

It is an object of the present invention to further improve a generic injection needle assembly according to the preamble of claim 1 such that holding arrangement of a needle tube, structure and operability of the injection needle assembly are improved.

The object of the present invention is achieved by an injection needle assembly having the features of claim 1.

Further advantageous developments of the invention are defined in the dependent claims.

Claim 8 discloses a drug injection device comprising an injection needle assembly according the present invention and a syringe.

It is an advantage of the present invention to provide an injection needle assembly and a drug injection device in which the needle tube can be reliably held by a chuck member, and the needle hub and the needle tube can be integrally formed by insert molding.

With the injection needle assembly and the drug injection device, by forming the retention hole into which the chuck member for holding and fixing the circumferential surface of the needle tube to the needle hub is inserted, it is possible to hold the needle tube not only at the both ends thereof in the axial direction but also from the circumferential surface thereof. As a result, since the needle tube can be reliably held by the chuck member, it is possible to integrally form the needle tube and the needle hub by insert molding.

Thus, since it is possible to integrally form the needle tube and the needle hub by insert molding, the step of adhering the needle tube and the needle hub to each other can be omitted, and therefore production cost can be reduced. Further, since even the circumferential surface of the needle tube can be held, the needle tube can be prevented from being deformed and/or bent due to the molding pressure generated when performing insert molding.

### Brief Description of Drawings

FIG. 1 is a side view showing a drug injection device according to an embodiment of the present invention;
FIG. 2 is an exploded view of the drug injection device according to the aforesaid embodiment;
FIG. 3 is a cross section of an injection needle assembly according to the aforesaid embodiment;
FIG. 4 is an exploded perspective view of the injection needle assembly according to the aforesaid embodiment; and
FIG. 5 is a side view showing a first member and a needle tube of the injection needle assembly according to the aforesaid embodiment;

### Best Modes for Carrying Out the Invention

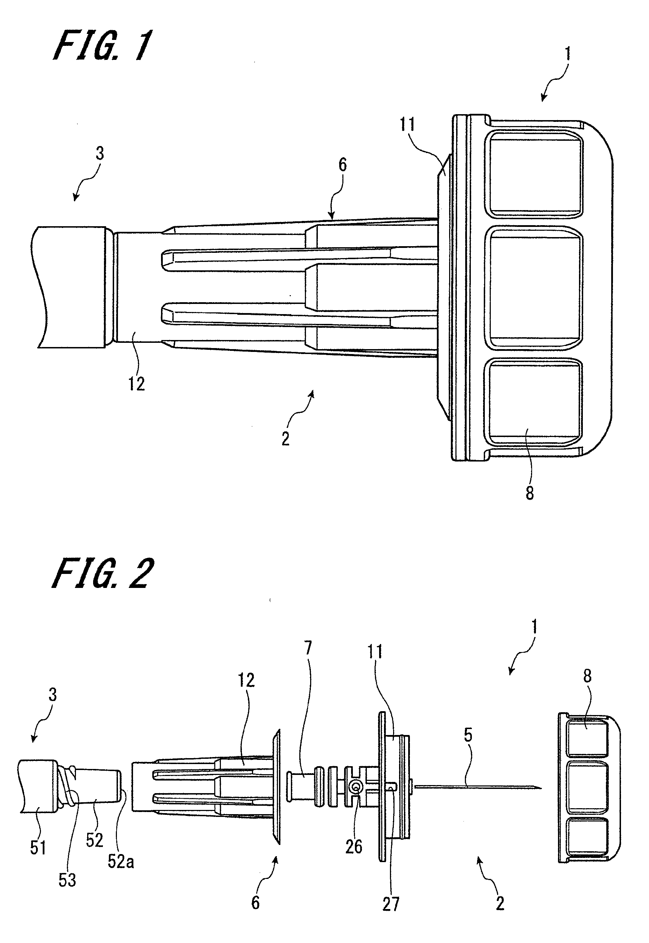

An injection needle assembly and a drug injection device according to an embodiment of the present invention will be described below with reference to FIGS. 1 to 5. Note that, in the drawings, like components are denoted by like numerals. Further, the present invention is not limited to the embodiment described below. The description will be given in the following order.
1. Configuration examples of injection needle assembly and drug injection device
2. Assembly of injection needle assembly and drug injection device
   2-1. Assembly of injection needle assembly
   2-2. Assembly of drug injection device
3. Method of using drug injection device

### 1. Configuration examples of injection needle assembly and drug injection device

### [Drug injection device]

An injection needle assembly and a drug injection device according to an embodiment (referred to as "the present embodiment" hereinafter) of the present invention will be described below with reference to FIG. 1 and FIG. 2.

FIG. 1 is a side view of the drug injection device of the present embodiment, and FIG. 2 is an exploded view of the drug injection device of the present embodiment.

A drug injection device 1 is used for sticking a needle tip into the skin from the skin surface to thereby inject a drug into the upper layer of the skin. The drug injection device 1 includes an injection needle assembly 2, and a syringe 3 detachably connected to the injection needle assembly 2.

Skin is composed of three layers: epidermis, dermis and subcutaneous tissue. The epidermis is a layer of about 50-200 µm from the skin surface, and the dermis is a layer of about 1.5-3.5 mm continuing from the epidermis. Since influenza vaccine is generally subcutaneously or intramuscularly administered, it is administered to the lower layer of the skin or the portion deeper than the lower layer.

On the other hand, it is considered to reduce the dose of the influenza vaccine by administering the influenza vaccine to the upper layer of skin as a target site where many immunocompetent cells are present. Incidentally, the upper layer of skin refers to the epidermis and the dermis of the skin.

As shown in FIG. 2, the injection needle assembly 2 includes a hollow needle tube 5 having a needle hole, a needle hub 6 to which the needle tube 5 is fixed, an elastic member 7 arranged in the needle hub 6, and a cap 8 detachably attached to the needle hub 6. Further, the needle hub 6 includes a first member 11, which is a holding portion for holding the needle tube 5, and a second member 12, which is a connecting portion to which the syringe 3 is connected.

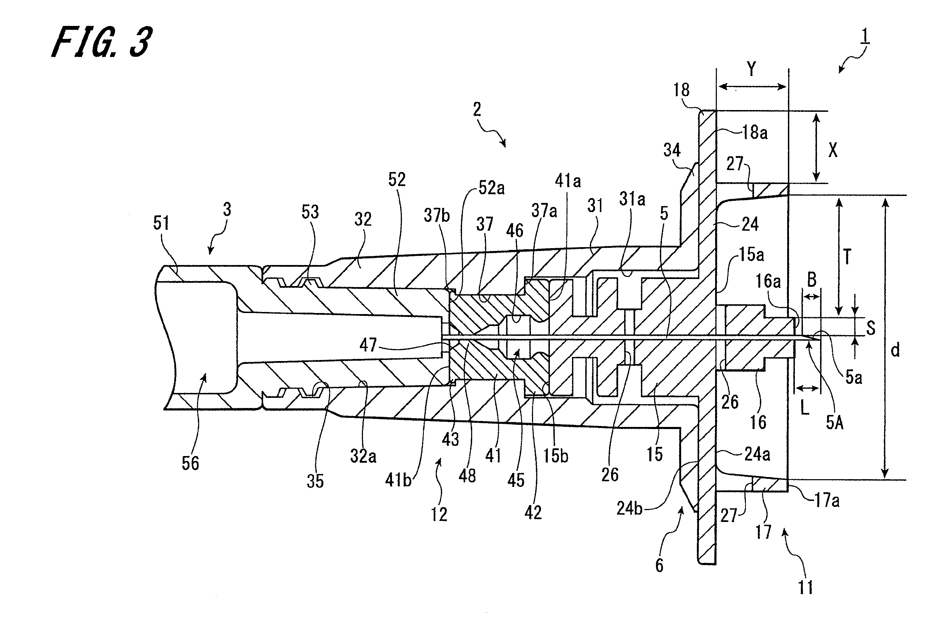

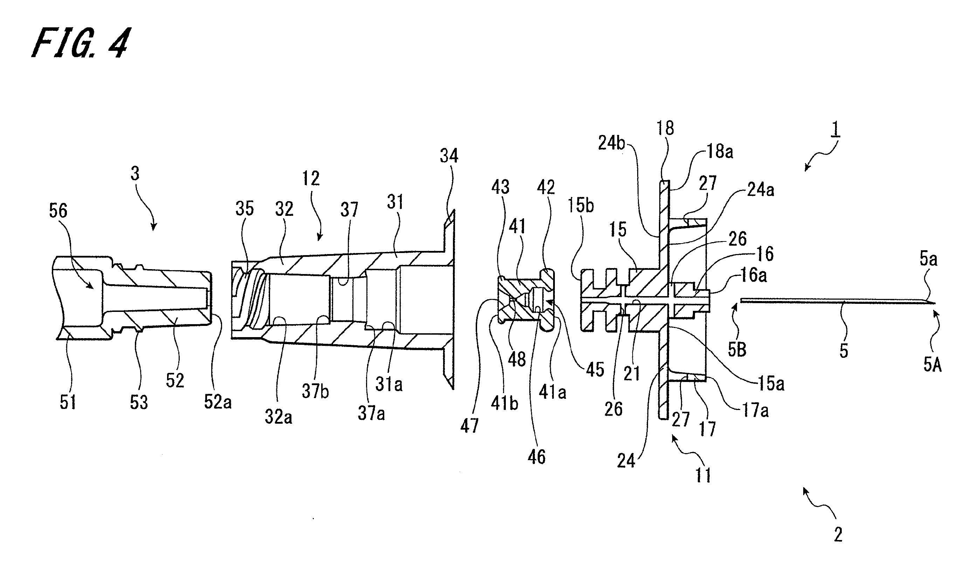

Next, the aforesaid components of the drug injection device 1 will be described below with reference to FIGS. 3 and 4.

FIG. 3 is a cross section of the drug injection device 1. FIG. 4 is an exploded perspective view of the drug injection device 1.

### [Injection needle assembly]

A needle tube of 22-33 gauge (outer diameter: 0.2-0.7 mm) according to ISO standard for medical needle tubes (IS09626: 1991/Amd. 1:2001(E)) can be used as the needle tube 5 of the injection needle assembly 2. Incidentally, when being used to perform administration to the upper layer of the skin, a needle tube of 26-33 gauge, preferably 30-33 gauge, can be used as the needle tube 5 of the injection needle assembly 2.

A needle tip 5A having a blade face 5a is provided on one end of the needle tube 5. The other end of the needle tube 5, on the side opposite to the needle tip 5A, will be referred to as a "base end 5B" hereinafter. The length of the blade face 5a in the axial direction of the needle tube 5 (referred to as "bevel length B" hereinafter) may be equal to or less than 1.4 mm (which is the minimum thickness of the upper layer of the skin of an adult, which is to be described later), but equal to or greater than about 0.5 mm, which is the bevel length when a short bevel is formed in a needle tube of 33 gauge. In other words, it is preferred that the bevel length B is set in a range of 0.5-1.4 mm.

It is further preferred that the bevel length B is equal to or less than 0.9 mm (which is the minimum thickness of the upper layer of the skin of a child), i.e., it is further preferred that the bevel length B is set in a range of 0.5-0.9 mm. Incidentally, the term "short bevel" means a blade face forming an angle of 18-25° with respect to the longitudinal direction of the needle, which is generally used as an injection needle. Further, a coating agent, such as silicone resin, fluorine resin or the like, is applied to the surface of the needle tip 5A of the needle tube 5. Therefore, it is possible to reduce the friction between the skin and the needle tube caused when puncturing the living body with the needle tube 5, so that it is possible to reduce the pain caused by the puncture.

The material of the needle tube 5 may be, for example, a stainless steel; however, instead of being limited to the stainless steel, the material of the needle tube 5 may also be other metal such as aluminum, aluminum alloy, titanium, titanium alloy, or the like. Further, not only a straight needle, but also a tapered needle in which at least a portion thereof is tapered may be used as the needle tube 5. The tapered needle may have a configuration in which the diameter of a base end portion of the needle is larger than the diameter of a tip end portion of the needle, and a middle portion of the needle has a taper structure. Further, the shape of the cross section of the needle tube 5 may also be a polygonal shape such as a triangular shape, instead of being limited to a circular shape. The needle tube 5 is fixed to the needle hub 6.

### [Needle hub]

Next, the needle hub 6 will be described below. The first member 11 and the second member 12 of the needle hub 6 are formed separately from each other; however, the first member 11 and the second member 12 may also be integrally formed. Examples of the material of the first member 11 and the second member 12 include synthetic resin such as polycarbonate, polypropylene, polyethylene or the like.

The first member 11 includes a substantially cylindrical base portion 15, an adjusting portion 16, a stabilizer 17, and a guide portion 18. The base portion 15 has two end faces 15a, 15b each perpendicular to the axial direction. The adjusting portion 16 is arranged in the central portion of the end face 15a on one end side in the axial direction of the base portion 15, and is formed as a cylindrical projection projected in the axial direction of the base portion 15. The axis of the adjusting portion 16 and the axis of the base portion 15 coincide with each other.

The base portion 15 and the adjusting portion 16 are configured so that the needle tube 5 penetrates through the axes of the base portion 15 and the adjusting portion 16. In other words, the needle tube 5 is formed integrally with the first member 11 by insert molding. Incidentally, there is another possible configuration in which, after the needle tube 5 and the first member 11 is integrally formed by insert molding, an adhesive is injected into the clearance between the needle tube 5 and the first member, so that the needle tube 5 and the first member 11 are reliably fixed to each other.

Further, the adjusting portion 16 is formed with a retention hole 26 that communicates with the needle tube 5 from the circumferential surface. The retention hole 26 is a through-hole perpendicular to the axis of the adjusting portion 16. A chuck member for holding and fixing the circumferential surface of the needle tube 5 is inserted into the retention hole 26. With such arrangement, when performing insert molding, the needle tube 5 can be held not only at the both ends in the axial direction, but also from the circumferential surface in the direction perpendicular to the axial direction, so that it is possible to easily perform the insert molding in a state where the needle tube 5 is stable. As a result, the needle tube 5 can be prevented from being deformed and/or bent due to the molding pressure.

The side of the base end 5B of the needle tube 5 is protruded from the end face 15b, which is the other end in the axial direction of the base portion 15. The base portion 15 is inserted into the second member 12 from the side of the end face 15b, and the side of the base end 5B of the needle tube 5 is inserted through an insertion hole 45 (which is to be described later) of the elastic member 7. The end face 15b of the base portion 15 abuts an end face 41a (which is to be described later) of the elastic member 7.

A connection piece 24 is provided on the outer circumferential surface of the base portion 15. The connection piece 24 is formed as a ring-like flange protruded outward in the radial direction of the base portion 15, and includes two flat surfaces 24a, 24b opposed to each other in the axial direction of the base portion 15. The second member 12 is connected to the flat surface 24b of the connection piece 24. The tip-end portion of the connection piece 24 is the guide portion 18. The details of the guide portion 18 will be described later.

Further, similar to the adjusting portion 16, the base portion 15 is also formed with a retention hole 26 into which a chuck member is inserted. Incidentally, although the present embodiment is described based on an example in which two retention holes 26 are formed, the number of the retention hole 26 may also be one, three or more than three, instead of being limited to two. In the case where a plurality of retention holes are formed, it is preferred that the plurality of retention holes are formed substantially at a predetermined interval in the first member 11 along the axial direction of the needle tube.

Further, in the case where one retention hole is formed, it is preferred that the retention hole is formed substantially at the center of the first member 11 in the axial direction of the needle tube, so that the needle tube 5 can be held in good balance. However, in the case where one retention hole is formed and where the length of the needle tube protruded from one side of the first member 11 is different from the length of the needle tube protruded from the other side of the first member 11 in the axial direction, it is preferred that the retention hole is arranged between the end of the first member 11 on the side from which the length of the needle tube protruded is smaller and the center of the needle tube in the axial direction.

The end face of the adjusting portion 16 is a needle-protruding surface 16a from which the side of the needle tip 5A of the needle tube 5 is protruded. The needle-protruding surface 16a is formed as a flat surface perpendicular to the axial direction of the needle tube 5. When the needle tube 5 is stuck into the upper layer of the skin, the needle-protruding surface 16a contacts the surface of the skin, so that the insertion depth of the needle tube 5 into the upper layer of the skin is regulated. In other words, the insertion depth of the needle tube 5 into the upper layer of the skin is determined by the length of the needle tube 5 protruding from the needle-protruding surface 16a (such length will be referred to as "protruding length L" hereinafter).

The thickness of the upper layer of the skin corresponds to the depth from the surface of the skin to the dermis layer, which is generally in a range of 0.5-3.0 mm. Therefore, the protruding length L of the needle tube 5 can be set in a range of 0.5-3.0 mm.

The vaccine is generally administered to the upper arm; however, when considering administering the vaccine to the upper layer of the skin, more appropriate portion to be stuck will be the portion around the shoulder where the skin is thick, especially the portion of the deltoid muscle. Therefore, the thickness of the upper layer of the skin overlying the deltoid muscle was measured for 19 children and 31 adults. The measurements were performed by imaging the upper layer of skin having high ultrasonic reflectivity using an ultrasonic measurement device (NP60R-UBM High Resolution Echo for Small Animal, NEPA GENE, CO., LTD.). Incidentally, since the measured values showed lognormal distribution, the range of MEAN ± 2SD was obtained by taking the geometrical mean.

The results showed that the thickness of the upper layer of skin overlying the deltoid muscle of a child was 0.9-1.6 mm. Further, the results also showed that the thickness of the upper layer of skin overlying the deltoid muscle of an adult was 1.4-2.6 mm in the distal portion, 1.4-2.5 mm in the middle portion, and 1.5-2.5 mm in the proximal portion. It can be confirmed from the above that the thickness of the upper layer of skin overlying the deltoid muscle is equal to or more than 0.9 mm for children, and is equal to or more than 1.4 mm for adults. Consequently, when performing injection on the upper layer of skin overlying the deltoid muscle, it is preferred that the protruding length L of the needle tube 5 is set in a range of 0.9-1.4 mm.

By setting the protruding length L in such a manner, it becomes possible to securely position the blade face 5a of the needle tip 5A in the upper layer of the skin. As a result, the needle hole (the drug solution outlet) opened on the blade face 5a can be positioned in the upper layer of the skin, regardless of its position in the blade face 5a. Incidentally, even when the drug solution outlet is positioned in the upper layer of the skin, if the needle tip 5A is deeply inserted into the upper layer of the skin, the drug solution will flow into the subcutaneous layer from between the side surface of the end portion of the needle tip 5A and the incised skin, and therefore it is important that the blade face 5a is securely positioned in the upper layer of the skin.

Incidentally, when being used to perform administration to the upper layer of the skin, it is difficult to make the bevel length B 1.0 mm or less if the needle tube is larger than 26 gauge. Accordingly, to set the protruding length L of the needle tube 5 in the preferable range (i.e., the range of 0.9-1.4 mm), it is preferred to use a needle tube smaller than 26 gauge.

The needle-protruding surface 16a is formed such that the distance S from the circumferential edge of the needle-protruding surface 16a to the circumferential surface of the needle tube 5 is 1.4 mm or less, preferably in a range of 0.3-1.4 mm. The distance S from the circumferential edge of the needle-protruding surface 16a to the circumferential surface of the needle tube 5 is set considering that a pressure will be applied to the blister formed by administering drug to the upper layer of the skin. In other words, the diameter of the needle-protruding surface 16a is set to sufficiently smaller than the diameter of the blister to be formed in the upper layer of skin, so that the formation of the blister will not be obstructed. As a result, even if the needle-protruding surface 16a presses the skin around the needle tube 5, the administered drug can be prevented from being leaked out.

The stabilizer 17 is formed in a tubular shape protruded from the flat surface 24a of the connection piece 24 provided in the base portion 15. The needle tube 5 and the adjusting portion 16 are arranged in the tube hole of the stabilizer 17. In other words, the stabilizer 17 is formed in a tubular shape covering around the adjusting portion 16 through which the needle tube 5 is penetrated, and is radially spaced from the needle tip 5A of the needle tube 5.

Further, the stabilizer 17 is provided with a cut-out 27 at a position corresponding to the retention hole 26 provided in the adjusting portion 16. Due to the provision of the cut-out 27, when performing insert molding, the stabilizer 17 and the chuck member can be prevented from interfering with each other, so that the operation of the chuck member can be secured.

The cap 8 is detachably fitted to the stabilizer 17 (see FIG. 1). The cap 8 covers the needle tip 5A of the needle tube 5. With such arrangement, when mounting the needle hub 6 on the syringe 3, the needle tip 5A can be prevented from being touched by the fingertips and the like of the user. Further, it is possible to constantly keep the used drug injection device 1 or injection needle assembly 2 in a safe condition, and it is possible for the user to dispose the used drug injection device 1 or injection needle assembly 2 without anxiety.

As shown in FIG. 3, an end face 17a of the stabilizer 17 is located closer to the side of the base end 5B of the needle tube 5 than the needle-protruding surface 16a of the adjusting portion 16. When the needle tip 5A of the needle tube 5 sticks the living body, the needle-protruding surface 16a first contacts the surface of the skin, and then the end face 17a of the stabilizer 17 contacts the surface of the skin. At this time, the end face 17a of the stabilizer 17 contacts the surface of the skin, and thereby the drug injection device 1 becomes stable, so that the needle tube 5 can be kept in an attitude where the needle tube 5 is substantially perpendicular to the skin.

Incidentally, the needle tube 5 can be kept in an attitude where the needle tube 5 is substantially perpendicular to the skin even if the end face 17a of the stabilizer 17 is coplanar with the needle-protruding surface 16a or located closer to the side of the needle tip 5A of the needle tube 5 than the needle-protruding surface 16a. Incidentally, considering the raised portion of the skin formed when the stabilizer 17 is pressed against the skin, it is preferred that the distance between the end face 17a of the stabilizer 17 and the needle-protruding surface 16a in the axial direction is set to 1.3 mm or less.

Further, the inner diameter d of the stabilizer 17 is set to a value equal to or larger than the diameter of the blister formed in the skin. To be specific, the distance T between the inner wall of the stabilizer 17 and the circumferential edge of the needle-protruding surface 16a is set in a range of 4-15 mm. With such arrangement, it is possible to prevent the possibility that the formation of the blister is obstructed by the pressure applied from the inner wall of the stabilizer 17 to the blister.

The minimal distance T between the inner wall of the stabilizer 17 and the outer circumferential surface of the adjusting portion 16 is set to 4 mm or more, but without upper limit. However, if the distance T is too large, the outer diameter of the stabilizer 17 will become large, and therefore it will be difficult to bring the entire end face 17a of the stabilizer 17 into contact with the skin when the needle tube 5 is to be stuck into the skin of a slender arm, such as an arm a child. Thus, considering the slender arms of children, it is preferred that the distance T is defined to 15 mm as the maximum.

Further, if the distance S between the circumferential edge of needle-protruding surface 16a and the circumferential surface of the needle tube 5 is 0.3 mm or more, the adjusting portion 16 will not enter the skin. Thus, considering the diameter (about 0.3 mm) of the needle-protruding surface 16a and the distance T (4 mm or more) between the inner wall of the stabilizer 17 and the circumferential edge of the needle-protruding surface 16a, the inner diameter d of the stabilizer 17 can be set to 9 mm or larger.

Incidentally, the shape of the stabilizer 17 is not limited to the circular tube shape, but may also be formed, for example, in a polygonal prism shape, such as a quadrangular prism shape, a hexagonal prism shape or the like, having a tube hole formed in the center thereof.

The guide portion 18 is a tip end portion of the connection piece 24 located outside in the radial direction of the first member 11 from the stabilizer 17. The guide portion 18 has a contact surface 18a that contacts the skin. The contact surface 18a is a portion of the flat surface 24a of the connection piece 24, and is a flat surface substantially parallel to the end face 17a of the stabilizer 17. By pressing the stabilizer 17 until the contact surface 18a of the guide portion 18 contacts the skin, the force applied from both the stabilizer 17 and the needle tube 5 for pressing the skin can be constantly maintained at or above a predetermined value, and thereby the protruded portion of the needle tube 5 from the needle-protruding surface 16a (corresponding to the protruding length L) can be securely inserted into the skin.

The length of the distance Y between the contact surface 18a of the guide portion 18 and the end face 17a of the stabilizer 17 (referred to as "guide portion height") is set so that the skin can be pressed and punctured by a suitable pressing force applied from the needle tube 5 and the stabilizer 17. Incidentally, the suitable pressing force of the needle tube 5 and the stabilizer 17 is in a range of, for example, 3N to 20N. As a result, the pressing force applied from the needle tube 5 and the stabilizer 17 to the skin is guided to the user by the guide portion 18, so that the needle tip 5A (the blade face 5a) of the needle tube 5 can be securely positioned in the upper layer of the skin, and it is possible to bring the user a sense of reassurance.

The guide portion height Y is suitably determined based on the inner diameter d of the stabilizer 17 and the length (referred to as "guide portion length" hereinafter) X between the tip end face of the guide portion 18 and the outer circumferential surface of the stabilizer 17. For example, when the inner diameter d of the stabilizer 17 is 12 mm and the guide portion length X is 3.0 mm, the guide portion height Y will be set in a range of 2.3-6.6 mm.

Next, the second member 12 will be described below. The second member 12 is substantially formed in a tubular shape. An end portion in the axial direction of the second member 12 is an insertion portion 31 into which the base portion 15 of the first member 11 is to be inserted, and the other end portion in the axial direction of the second member 12 is a fitting portion 32 for fitting a discharge portion 52 (which is to be described later) of the syringe 3 therein. The size of a tube hole 31a of the insertion portion 31 is set corresponding to the size of the base portion 15 of the first member 11.

The insertion portion 31 is provided with a fixing piece 34, to which the connection piece 24 of the first member 11 is connected. The fixing piece 34 is a ring-like flange formed continuously from the tip end of the insertion portion 31 and protruded outward in the radial direction. The flat surface 24b of the connection piece 24 provided in the first member 11 abuts the fixing piece 34 so as to be fixed. Examples of the method for fixing the fixing piece 34 and the connection piece 24 to each other include: adhesive, ultrasonic welding, laser welding, screw fixation and the like.

The diameter of a tube hole 32a of the fitting portion 32 is set corresponding to the diameter of the discharge portion 52 of the syringe 3, and continuously deceases as going toward the side of the insertion portion 31. A thread groove 35 for screwing the discharge portion 52 of the syringe 3 is formed in the inner surface of the fitting portion 32.

An engaging portion 37, to which the elastic member 7 is engaged, is provided between the insertion portion 31 and the fitting portion 32. The engaging portion 37 is formed as a stepped portion protruded inward in the radial direction from the inner surface of the second member 12, and has two engaging surfaces 37a, 37b each substantially perpendicular to the axial direction of the second member 12. A flange portion 42 (which is to be described later) of the elastic member 7 is engaged with the engaging surface 37a of the engaging portion 37, and a stopper projection 43 of the elastic member 7 is engaged with the engaging surface 37b of the engaging portion 37.

### [Elastic member]

Next, the elastic member 7 will be described below. The elastic member 7 is arranged inside the second member 12 of the needle hub 6, and is interposed between the first member 11 and the syringe 3. The elastic member 7 includes a body portion 41, the flange portion 42 provided on one end in the axial direction of the body portion 41, and the stopper projection 43 provided the other end of the body portion 41.

The body portion 41 is formed in a substantially cylindrical shape, and has two end faces 41a, 41b each perpendicular to the axial direction. The end face 15b of the base portion 15 of the first member 11 abuts the end face 41a of the body portion 41, and the tip end of the discharge portion 52 of the syringe 3 liquid-tightly abuts the end face 41b of the body portion 41. In other words, the end face 41b is an abutting surface on which the tip end of the discharge portion 52 liquid-tightly abuts.

The body portion 41 is formed with an insertion hole 45 through which the side of the base end 5B of the needle tube 5 protruded from the end face 15b of the base portion 15 is inserted. The insertion hole 45 extends in the axial direction of the body portion 41, and is opened on both end faces 41a, 41b. The inner surface of body portion 41 is defined by an end face side separated portion 46, an abutting surface side separated portion 47, and an adhesive portion 48.

The end face side separated portion 46 forms the opening of the insertion hole 45 on the end face 41a. The end face side separated portion 46 is spaced from the outer circumferential surface of the needle tube 5, and is formed in a tapered shape so that the diameter of the insertion hole 45 continuously increases as going toward the side of the end face 41a. With such arrangement, the side of the base end 5B of the needle tube 5 protruded from the end face 15b of the base portion 15 can be easily inserted through the insertion hole 45. Incidentally, the end face side separated portion 46 of the insertion hole 45 may also be formed in other shapes, instead of being limited to the tapered shape, as long as such shapes enable the needle tube 5 to be easily inserted through the insertion hole 45.

The abutting surface side separated portion 47 forms the opening of the insertion hole 45 on the end face 41b. The abutting surface side separated portion 47 is spaced from the outer circumferential surface of the needle tube 5, and is formed in a tapered shape so that the diameter of the insertion hole 45 continuously increases as going toward the side of the end face 41b. By providing the abutting surface side separated portion 47 to the elastic member 7, the side of the end face 41b of the body portion 41 is elastically deformed so as to cover the base end 5B of the needle tube 5, so that the needle hole can be prevented from being closed.

Further, instead of being limited to the tapered shape, the abutting surface side separated portion 47 of the insertion hole 45 may also be formed as, for example, a recessed portion which has a diameter larger than the diameter of the adhesive portion 48 and which is spaced from the outer circumferential surface of the needle tube 5. In other words, the abutting surface side separated portion 47 of the insertion hole 45 may also be formed in other shapes as long as such shapes enable the side of the end face 41b of the body portion 41 to be elastically deformed so as to cover the base end 5B of the needle tube 5, so that the needle hole can be prevented from being closed.

The adhesive portion 48 is formed between the end face side separated portion 46 and the abutting surface side separated portion 47. The adhesive portion 48 adheres liquid-tightly to the outer circumferential surface of the needle tube 5. With such arrangement, the drug inside the syringe 3 can be prevented from being penetrated into the side of the first member 11 of the needle hub 6 from between the needle tube 5 and the elastic member 7.

The flange portion 42 is formed in a ring-like shape protruded outward in the radial direction from the outer circumferential surface of the body portion 41. The outer diameter of the flange portion 42 is substantially equal to the outer diameter of the base portion 15 of the first member 11. Thus, one flat surface of the flange portion 42 abuts the engaging surface 37a of the engaging portion 37 arranged in the second member 12, and the other flat surface of the flange portion 42 abuts the end face 15b of the base portion 15 of the first member 11. The elastic member 7 is attached to the needle hub 6 by sandwiching the flange portion 42 with the engaging portion 37 of the second member 12 and the base portion 15 of the first member 11.

Similar to the flange portion 42, the stopper projection 43 is formed in a ring-like shape protruded outward in the radial direction from the outer circumferential surface of the body portion 41. The stopper projection 43 is engaged with the engaging surface 37b of the engaging portion 37 provided in the second member 12. The flange portion 42 and the stopper projection 43 are engaged with the engaging portion 37 of the second member 11, and thereby the movement of the elastic member 7 in the axial direction is locked.

Examples of the material of the elastic member 7 include various kinds of rubber material (such as natural rubber, silicone rubber and the like), various kinds of thermoplastic elastomer (such as polyurethane elastomer, styrene elastomer and the like), and elastic materials each obtained by mixing the aforesaid materials.

### [Syringe]

The syringe 3 includes a flange body 51 and the discharge portion 52 formed continuously from the flange body 51. The flange body 51 is formed as a circular tube. The discharge portion 52 is protruded from one end in the axial direction of the flange body 51, and is formed as a circular tube having an outer diameter smaller than that of the flange body 51. The discharge portion 52 is formed in a tapered shape so that the diameter of the discharge portion 52 continuously decreases as going toward the side of the tip end. An end face 52a (i.e., the tip end) of the discharge portion 52 is a flat surface perpendicular to the axial direction, and liquid-tightly abuts the end face 41b of the elastic member 7. Further, a threaded portion 53 for screwing the second member 12 of the needle hub 6 is formed in the outer circumferential surface of the discharge portion 52.

A gasket (not shown) is housed in the flange body 51. The space inside the flange body 51 is divided into two parts by the gasket. One part of the space communicating with the discharge portion 52, along with the space inside the discharge portion 52, defines a liquid chamber 56. A plunger (not shown) is arranged in the other part of the space inside the flange body 51. The plunger is connected to the gasket, and is protruded from the opening on the other end of the flange body 51. By operating the plunger, the gasket is moved within the flange body 51 in the axial direction, and thereby aspiration of the drug into the liquid chamber 56 and discharge of the drug filled into the liquid chamber 56 are performed.

The material of the flange body 51 and the discharge portion 52 may be a synthetic resin (such as polycarbonate, polypropylene, polyethylene or the like), or a metal (such as stainless steel, aluminum or the like).

### 2. Assembly of injection needle assembly and drug injection device

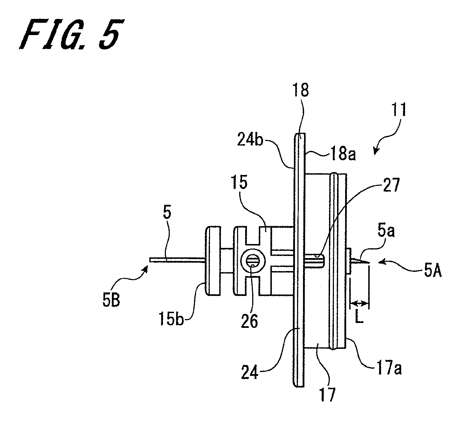

A method of assembling the injection needle assembly 2 and the drug injection device having the aforesaid configuration will be described below with reference to FIG. 5 and FIG. 3.

FIG. 5 is a side view showing a state where the first member 11 and the needle tube 5 is integrally formed.

### 2-1. Assembly of injection needle assembly

First, the needle tip 5A, base end 5B (which are the both ends in the axial direction of the needle tube 5) of the needle tube 5, and the side surface of the needle tube 5 are held and fixed by the chuck members. Next, the insert molding is performed to form the first member 11 around the needle tube 5. At this time, the length of the needle tip 5A of the needle tube 5 protruded from the first member 11 is set in a range of 0.9-1.4 mm, which is smaller than the length of the base end 5B of the needle tube 5 protruded from the first member 11. Further, the two retention hole 26, into which the chuck members are inserted, and the cut-out 27 are formed in the side surface of the first member 11. Thereby the step of attaching the needle tube 5 is completed; and in such state, the needle tube 5 is held by the first member 11.

With such arrangement, since the needle tube 5 and the first member 11 can be integrally formed by insert molding, the adhering step using adhesive can be omitted, so that the assembly process can be simplified. Incidentally, in order to more strongly fix the needle tube 5 and the first member 11 to each other, an adhesive may be injected from the retention hole 26 formed in the base portion 15.

Next, a coating agent, such as silicone resin, fluorine resin or the like, is coated on the needle tip 5A of the needle tube 5. At this time, the extra coating agent is infiltrated into the clearance between the needle tube 5 and the first member 11 by capillarity. However, the extra coating agent can be discharged to the outside from the clearance between the needle tube 5 and the first member 11 through the retention hole 26 formed in the adjusting portion 16.

Next, the second member 12, to which the elastic member 7 has been previously engaged, is connected to the first member 11 which holds the needle tube 5. In other words, the base portion 15 of the first member 11 and the side of the base end 5B of the needle tube 5 are inserted into the insertion portion 31 of the second member 12, so that the connection piece 24 of the first member 11 abuts the fixing piece 34 of the second member 12. At this time, the side of the base end 5B of the needle tube 5 is inserted through the insertion hole 45 of the elastic member 7 arranged inside the second member 12, and adhered liquid-tightly to the adhesive portion 48 (see FIG. 3). Thereafter, the fixing piece 34 of the second member 12 is fixed to the connection piece 24 of the first member 11 by a fixing method such as adhesive, ultrasonic welding, laser welding, screw fixation or the like. By the aforesaid steps, the assembly of the injection needle assembly 2 is completed.

Incidentally, the method of assembling the injection needle assembly 2 is not limited to the aforesaid method. For example, the first member 11 and the second member 12 may also be connected to each other after the elastic member 7 has been attached to the base end 5B of the needle tube 5 held on the first member 11.

### 2-2. Assembly of drug injection device

Next, the assembly of the drug injection device, i.e., the method of mounting the injection needle assembly 2 to the syringe 3, will be described below. In order to mount the injection needle assembly 2 to the syringe 3, first the discharge portion 52 of the syringe 3 is inserted into the fitting portion 32 of the injection needle assembly 2. Thereafter, the threaded portion 53 of the discharge portion 52 is screwed with the thread groove 35 of the fitting portion 32. By the aforesaid steps, the injection needle assembly 2 is mounted to the syringe 3, and thereby the assembly of the drug injection device 1 is completed.

### 3. Method of using drug injection device

Next, the method of using the drug injection device 1 will be described below. In order to stick the needle tip 5A of the needle tube 5 into the living body, first the end face 17a of the stabilizer 17 is caused to face the skin. Thereby the needle tip 5A of the needle tube 5 faces the skin to be stuck. Next, the drug injection device 1 is moved substantially perpendicular to the skin, so that the needle tip 5A is stuck into the skin while the end face 17a of the stabilizer 17 is pressed against the skin. At this time, the needle-protruding surface 16a contacts the skin so that the skin can be flatly deformed, while the needle tip 5A of the needle tube 5 can be inserted into the skin by a depth equivalent to the protruding length L.

Next, the end face 17a of the stabilizer 17 is pressed until the contact surface 18a of the guide portion 18 contacts the skin. Here, the value of the guide portion height y (see FIG. 3) is set so that the skin can be stuck by a suitable pressing force applied from the needle tube 5 and the stabilizer 17. Thus, the force of the stabilizer 17 for pressing against the skin becomes a predetermined value.

As a result, the suitable pressing force of the stabilizer 17 can be recognized by the user, and the needle tip 5A and the blade face 5a of the needle tube 5 can be securely positioned in the upper layer of the skin. In such a manner, the guide portion 18 becomes a landmark for recognizing the suitable pressing force of the stabilizer 17, and thereby the user can use the drug injection device 1 with a sense of reassurance.

Further, since the stabilizer 17 abuts the skin, the attitude of the drug injection device 1 becomes stable, so that it is possible to stick the needle tube 5 straight into the skin. Further, after the needle tip has been stuck into the skin, waggling caused in the needle tube 5 can be prevented, and therefore the drug can be administered stably. In the case of a needle tube with a very short protruding length of about 0.5 mm, for example, there is a possibility that the needle tip will not be inserted into the skin even if the needle tip is brought into contact with the skin. However, since the skin is pressed down perpendicularly by the stabilizer 17, the skin inside the stabilizer 17 is strained so as to be in a tensioned state. Thus, since the skin becomes hard to flee from the needle tip 5A of the needle tube 2, the stabilizer 17 has an effect of making it easier for the needle tip 5A to stick the skin.

After the side of the needle tip 5A of the needle tube 5 has stuck into the skin, the plunger (not shown) is pressed, so that the gasket (not shown) is moved toward the side of the discharge portion 52. Thus, the drug filled in the liquid chamber 56 of the syringe 3 is pushed out from the discharge portion 52 and injected into the upper layer of the skin from the needle tip 5A through the needle hole of the needle tube 5. At this time, since a space is formed between the tip end of the discharge portion 52 and the base end 5B of the needle tube 5, the residual amount of the drug can be reduced.

It is to be understood that the present invention is not limited to the embodiment described above and shown in the attached drawings, and various modifications can be made which fall under the scope of the present invention as defined in the appended claims. For example, although the present embodiment is described based on an example in which an elastic member is provided between the first member and the second member, the injection needle assembly may be configured without the elastic member, or the second member and the elastic member may be integrally formed.

### Explanation of Reference Numerals

- 1: drug injection device
- 2: injection needle assembly
- 3: syringe
- 5: needle tube
- 5A: needle tip
- 5b: base end
- 5a: blade face
- 6: needle hub
- 7: elastic member
- 11: first member
- 12: second member
- 15: base portion
- 16: adjusting portion
- 16a: needle-protruding surface
- 17: stabilizer
- 18: guide portion
- 24: connection piece
- 26: retention hole
- 27: cut-out
- L: protruding length
- S: distance from the circumferential edge of the needle-protruding surface to the circumferential surface of the needle tube
- T: distance between the inner wall of the stabilizer and the outer circumferential surface of the adjusting portion
- x: guide portion length
- y: guide portion height
- d: inner diameter

## Claims

1. An injection needle assembly (2) comprising:
a needle tube (5) having a needle tip (5A) capable of puncturing a living body; and
a needle hub (6) that is adapted to hold the needle tube (5) in a state where the needle tip (5A) of the needle tube (5) is protruded,
wherein the needle hub (6) has a stabilizer (17) arranged to cover around the needle tube (5) in a state where the needle tip (5A) of the needle tube (5) is protruded, the stabilizer (17) having an end face (17a) which contacts the skin when the needle tube (5) punctures the living body,
**characterized in that**
the needle hub (6) is formed with a retention hole (26) into which a chuck member is inserted, the chuck member being adapted to hold and fix the circumferential surface of the needle tube (5) when performing insert molding,
the retention hole (26) extends perpendicular to the axial direction of the needle tube (5) arranged in the needle hub (6), and
the stabilizer (17) is formed with a cut-out (27) at a position corresponding to the retention hole (26) for securing the operation of the chuck member.

2. The injection needle assembly (2) according to claim 1, wherein another retention hole (26) is formed substantially at the center of the needle hub (6) in the axial direction of the needle tube (5).

3. The injection needle assembly (2) according to claim 1 or 2, wherein the retention hole (26) comprises a plurality of retention holes (26) formed in the needle hub (6).

4. The injection needle assembly (2) according to claim 3, wherein the plurality of retention holes (26) are formed in the needle hub (6) substantially at a predetermined interval along the axial direction of the needle tube (5).

5. The injection needle assembly (2) according to any one of claims 1 to 4, wherein the needle hub (6) has an adjusting portion (16) arranged around the needle tube (5), the adjusting portion (16) having a needle-protruding surface (16a) from which the needle tip (5A) of the needle tube (5) is protruded.

6. The injection needle assembly (2) according to any one of claims 1 to 5, wherein the length of needle tip portion (5A) of the needle tube (5) protruded from the needle hub (6) is smaller than the length of needle tube (5) protruded from the side opposite to the needle hub (6).

7. The injection needle assembly (2) according to any one of claims 1 to 6, wherein the needle tube (5) has an outer diameter of 0,2 to 0,7mm (i.e. is in a range of 22 to 33 gauge).

8. A drug injection device (1) comprising:
an injection needle assembly (2) according any one of the claims 1 to 7; and
a syringe (3) connected to the needle hub (6).

## Patentansprüche

1. Injektionsnadelbaugruppe (2) mit:
einer Nadelkanüle (5) mit einer Nadelspitze (5A), die in der Lage ist, einen lebenden Körper zu punktieren; und
einer Nadelnabe (6), die angepasst ist, um die Nadelkanüle (5) in einem Zustand zu halten, in dem die Nadelspitze (5A) der Nadelkanüle (5) nach außen vorragt,
wobei die Nadelnabe (6) einen Stabilisator (17) hat, der angeordnet ist, um die Nadelkanüle (5) in einem Zustand zu überdecken, in dem die Nadelspitze (5A) der Nadelkanüle (5) nach außen vorragt, wobei der Stabilisator (17) eine Stirnfläche (17a) hat, die die Haut berührt, wenn die Nadelkanüle (5) den lebenden Körper punktiert,
**dadurch gekennzeichnet, dass**
die Nadelnabe (6) mit einem Retentionsloch (26) ausgebildet ist, in das ein Futterbauteil eingesetzt ist, wobei das Futterbauteil angepasst ist, um die Umfangsfläche der Nadelkanüle (5) zu halten und zu fixieren, wenn ein Umspritzen ausgeführt wird,
sich das Retentionsloch (26) senkrecht zu der axialen Richtung der Nadelkanüle (5) erstreckt, die in der Nadelnabe (6) angeordnet ist, und
der Stabilisator (17) mit einem Ausschnitt (27) an einer Position korrespondierend zu dem Retentionsloch (26) zum Sichern der Handhabung des Futterbauteils ausgebildet ist.

2. Injektionsnadelbaugruppe (2) nach Anspruch 1, wobei ein weiteres Retentionsloch (26) im Wesentlichen an der Mitte der Nadelnabe (6) in der axialen Richtung der Nadelkanüle (5) ausgebildet ist.

3. Injektionsnadelbaugruppe (2) nach Anspruch 1 oder 2, wobei das Retentionsloch (26) eine Vielzahl von Retentionslöchern (26) aufweist, die in der Nadelnabe (6) ausgebildet sind.

4. Injektionsnadelbaugruppe (2) nach Anspruch 3, wobei die Vielzahl von Retentionslöchern (26) in der Nadelnabe (6) im Wesentlichen in einem vorbestimmten Intervall entlang der axialen Richtung der Nadelkanüle (5) ausgebildet ist.

5. Injektionsnadelbaugruppe (2) nach einem der Ansprüche 1 bis 4, wobei die Nadelnabe (6) einen Einstellabschnitt (16) hat, der um die Nadelkanüle (5) herum angeordnet ist, wobei der Einstellabschnitt (16) eine Nadelvorragefläche (16a) hat, von der die Nadelspitze (5A) der Nadelkanüle (5) nach außen vorragt.

6. Injektionsnadelbaugruppe (2) nach einem der Ansprüche 1 bis 5, wobei die Länge eines Nadelspitzenabschnitts (5A) der Nadelkanüle (5), der von der Nadelnabe (6) vorragt, kleiner ist als die Länge der Nadelkanüle (5), die von der Seite entgegengesetzt zu der Nadelnabe (6) vorragt.

7. Injektionsnadelbaugruppe (2) nach einem der Ansprüche 1 bis 6, wobei die Nadelkanüle (5) einen Außendurchmesser von 0,2 bis 0,7 mm hat (d.h. in einem Bereich von 22 bis 33 Gauge liegt).

8. Arzneimittelinjektionsvorrichtung (1) mit:
einer Injektionsnadelbaugruppe (2) nach einem der Ansprüche 1 bis 7; und
einer Spritze (3), die mit der Nadelnabe (6) verbunden ist

## Revendications

1. Ensemble d'aiguille d'injection (2) comprenant :
un tube d'aiguille (5) ayant une pointe d'aiguille (5A) capable de perforer un corps vivant ; et
un pavillon d'aiguille (6) qui est adapté pour maintenir le tube d'aiguille (5) dans un état où la pointe d'aiguille (5A) du tube d'aiguille (5) fait saillie,
dans lequel le pavillon d'aiguille (6) a un stabilisateur (17) agencé pour effectuer une couverture autour du tube d'aiguille (5) dans un état dans lequel la pointe d'aiguille (5A) du tube d'aiguille (5) fait saillie, le stabilisateur (17) ayant une face d'extrémité (17a) qui vient en contact avec la peau lorsque le tube d'aiguille (5) perfore le corps vivant,
**caractérisé en ce que**
le pavillon d'aiguille (6) est formé d'un trou de retenue (26) dans lequel un élément de mandrin est inséré, l'élément de mandrin étant adapté pour maintenir et fixer la surface circonférentielle du tube d'aiguille (5) lors de la réalisation d'un moulage par insertion,
le trou de retenue (26) s'étend perpendiculairement à la direction axiale du tube d'aiguille (5) agencé dans le pavillon d'aiguille (6), et
le stabilisateur (17) est formé avec une découpe (27) à une position correspondant au trou de retenue (26) pour assurer le fonctionnement de l'élément de mandrin.

2. Ensemble d'aiguille d'injection (2) selon la revendication 1, dans lequel un autre trou de retenue (26) est formé substantiellement au centre du pavillon d'aiguille (6) dans la direction axiale du tube d'aiguille (5).

3. Ensemble d'aiguille d'injection (2) selon la revendication 1 ou 2, dans lequel le trou de retenue (26) comprend une pluralité de trous de retenue (26) formée dans le pavillon d'aiguille (6).

4. Ensemble d'aiguille d'injection (2) selon la revendication 3, dans lequel la pluralité de trous de retenue (26) sont formés dans le pavillon d'aiguille (6) substantiellement au niveau d'un intervalle prédéterminé le long de la direction axiale du tube d'aiguille (5).

5. Ensemble d'aiguille d'injection (2) selon l'une quelconque des revendications 1 à 4, dans lequel le pavillon d'aiguille (6) a une partie d'ajustement (16) agencée autour du tube d'aiguille (5), la partie d'ajustement (16) ayant une surface de saillie d'aiguille (16a) depuis laquelle la pointe d'aiguille (5A) du tube d'aiguille (5) fait saillie.

6. Ensemble d'aiguille d'injection (2) selon l'une quelconque des revendications 1 à 5, dans lequel la longueur de la partie de pointe d'aiguille (5A) du tube d'aiguille (5) faisant saillie depuis le pavillon d'aiguille (6) est inférieure à la longueur du tube d'aiguille (5) faisant saillie depuis le côté opposé au pavillon d'aiguille (6).

7. Ensemble d'aiguille d'injection (2) selon l'une quelconque des revendications 1 à 6, dans lequel le tube d'aiguille (5) a un diamètre externe allant de 0,2 à 0,7 mm (c'est-à-dire se trouve dans une plage allant de 22 à 33 jauges).

8. Dispositif d'injection de médicaments (1) comprenant :
un ensemble d'aiguille d'injection (2) selon l'une quelconque des revendications 1 à 7 ; et
une seringue (3) liée au pavillon d'aiguille (6).
